Europäisches Patentamt

European Patent Office

Office européen des brevets

(19)

(11) Publication number: **0 382 457**
**A2**

# EUROPEAN PATENT APPLICATION

(12)

(21) Application number: 90301193.0

(22) Date of filing: 05.02.90

(51) Int. Cl.5: **A61F 2/00, A61B 17/56,**
**A61L 25/00, A61L 27/00,**
**B05D 7/00**

(30) Priority: 06.02.89 US 307380

(43) Date of publication of application:
16.08.90 Bulletin 90/33

(84) Designated Contracting States:
AT BE CH DE DK ES FR GB GR IT LI LU NL SE

(71) Applicant: Pfizer Hospital Products Group,
Inc.
235 East 42nd Street
New York New York 10017(US)

(72) Inventor: Kenna, Robert V.
505 S. Beach Road
Hobe Sound, Florida(US)

(74) Representative: Wood, David John et al
PFIZER LIMITED, Ramsgate Road
Sandwich, Kent CT13 9NJ(GB)

(54) Method and apparatus for improvement of bone healing.

(57) A method and apparatus 10 are provided for improving bone ingrowth into prostheses and for stimulating living bone attachment onto prostheses and for bone healing in general wherever two bone surfaces are to be joined, for example in fractures and in osteotomies. Bone particles are sprayed under particular conditions and with a particular device 10 onto the two surfaces to be joined. The device 10 provides a fine and controlled spray of bone particles when the device is activated, without waste of valuable bone and without clogging of the gun 10 by relatively sticky and moist bone particles.

Fig. 1.

EP 0 382 457 A2

## METHOD AND APPARATUS FOR IMPROVEMENT OF BONE HEALING

This invention relates generally to bone growth and to apparatus suitable for facilitating such growth and relates in particular to a method and apparatus for depositing bone particles onto bone surfaces and onto prostheses which are to be implanted into bone.

An area of continued interest and research is for methods to improve the healing process wherever bones are involved. This includes implanting prostheses into bones, healing of fractures, and healing of cut surfaces of bone (osteotomies).

In the prior art, in U.S. patent 3,918,100 to Shaw et al. which issued on November 11, 1975, the technique of rf sputtering was applied to the problem of forming a bone replacement or bone repair prosthesis. Bone was sputtered onto prostheses to form a covering which stimulated bone growth and attachment to natural bone.

However, despite that early work, there has been a continuing need for improved methods of stimulating bone healing.

Also, although air brushes have been used in spraying of paint, a device suitable for use in spraying relatively wet, sticky solid bone particles has not been known.

An object of this invention is a method for improving bone healing in any surgical technique that requires the healing process, including techniques wherein prosthetic devices are implanted into bone, healing of fractures, and healing of cut surfaces of bone (as in osteotomies).

Another object of this invention is a method for improving bone ingrowth into prosthetic devices, including implants having either porous and/or smooth surfaces.

Yet another object of this invention is an apparatus which is especially useful for spraying sticky, relatively wet material, for example bone onto either bone or implant surfaces, so as to promote bone growth.

According to the invention, a method for improving bone healing between two surfaces of bone (for example, in fractures or osteotomies) and for improving bone growth between bone and prosthetic device implanted into that bone comprises spraying particles of bone which have retained their growth factor (i.e., either autogenous bone, which has been removed from the patient at the time of surgery and which is alive, or allegraft bone, which has been obtained from a bone bank and which has retained some growth factor although it may not be alive) onto the surfaces on which bone ingrowth or bone healing is to take place, under conditions such that the bone retains its growth factor. The bone is milled (if necessary by hand) such that the temperature of the bone is kept below a temperature which would kill living bone; and the bone is not sprayed with any gas (e.g. nitrogen) that would kill the bone.

For autogenous and allegraft bone, a suitable temperature for effective spraying lies within the range from about 50 to about 100° F; and clean, filtered compressed air is used for spraying at a pressure between about 30 and about 80 p.s.i.

The sizes of the bone particles will generally be such that they have a largest dimension which lies within the range from about 0.3 to about 1.5 mm.

Also according to the invention, an apparatus which is especially suitable for spraying a measured amount of relatively wet solid particles (e.g., bone) in the method of the invention comprises in operable communications:

(a) a housing having a first aperture located within that housing for allowing a gas jet to exit from the housing;

(b) means for holding a relatively large amount of the particles in proximity to a second aperture spaced apart from the first aperture, wherein intake air passes through the relatively large amount of the particles and into the housing through the second aperture when the second aperture is in open communication with the atmosphere and is an open position;

(c) means for separating off a relatively small amount of the particles from the relatively large amount of the particles;

(d) means for placing the relatively small amount of the particles and the first aperture into proximity with one another, such that the second aperture is in a closed position and is not longer in open communication with the atmosphere.

In a preferred embodiment, the first aperture of item (a) allows a gas jet to continuously exit from the housing.

In another preferred embodiment, the means for separating off a small amount of particles is a means for slicing.

In the practice of the invention, either autogenous bone (which has been removed from a patient and is still alive) or allegraft bone (which has been obtained ·from a bone bank and still retains some growth factor, although it is not alive) can be used in the method of the invention for improving bone growth into prostheses, for stimulating living bone attachment onto prostheses, and for bone healing in general whereever two bone surfaces are to be joined. Bone particles are sprayed under particular conditions, as described below.

It is very important that the bone remain at relatively low temperatures, and not be subjected to temperatures higher than about 100° F. The bone should remain within the range from about 50 to about 100° F (and the procedures for preparation and use of the bone particles will be preferably at a temperature of about 70° F).

The gas which is used to spray the bone particles will preferably be clean and filtered compressed air. It has been found that nitrogen gas kills living bone, and therefore nitrogen should not be selected as the type of compressed gas for spraying the bone particles. The gas which is selected should be clean and filtered. The pressure of the gas should lie within the range from about 30 to about 80 p.s.i., and will preferably be within the range from about 50 to about 70 p.s.i.

The sizes of the particles of bone which are sprayed either onto an implant (when it is desired that bone will attach onto a prosthesis or grow into a prosthesis or when bone healing between two bone surfaces is desired) will generally be such that the largest dimension of the bone particles lie within the range from about 0.3 to about 1.5 mm. It has been generally found that the smaller the size of the bone particles, the faster they tend to grow; hence, particles as small as 100 microns can be used for spraying.

The finely powdered bone particles are used for spraying. The larger sized particles can be packed either onto an implant or onto two bone surfaces to be joined (followed if desired by spraying a layer of bone cement as a final layer). Bone shavings also can be finger packed, if desired.

The bone particles are prepared in the following manner. First, any cartilage present is removed by any suitable method. Next, the bone is ground in a bone mill in such a manner that the temperature range does not exceed about 100° F, as described above. Thus, the bone should either be ground by hand or by use of a device which does not introduce any significant amount of heat into the bone. Next, the ground bone particles are sprayed either onto bone-bone surfaces or onto bone-prosthesis surfaces, using a suitable device, such that the bone particles are sprayed in a fine and controlled spray, without waste of valuable bone and without clogging of the device. Such a suitable bone spray gun is described and claimed herein. Larger sized bone particles can, if desired, be finger packed into bone-bone junctures or into various implant surfaces, described below, either with or without bone cement. Additionally, a top layer of bone cement can be applied, if desired.

It is desirable to reinforce growth factors of the bone so as to encourage growth of living bone into either bone-bone junctures or into prostheses which have been implanted into bone. The fine spray of bone material appears to act as a nutrient for immature calcified tissue.

When it is desired to have bone ingrowth into an implant, either smooth or porous implants can be used. However, it is believed that best results are obtained when the bone particles have a size smaller than the pore size of the implant. Additionally, for such bone ingrowth into an implant, the implant should have an undercut (i.e., some type of interlocking surface). Very fine particles of bone are used for spraying applications, and less fine particles of bone are used for packing applications (for example, to fill in any large spaces).

Preferably, bone will first be sprayed onto all surfaces, followed by any finger packing as desired.

The bone particles can be sprayed onto any surfaces that requires the healing process, for example, implants into which it is desired that bone grow, fractures, or cut surfaces of bone (i.e., osteotomies).

If desired, bone cement can be placed onto the larger bone particles or shavings for packing; however, the method of bone ingrowth as described above is not generally intended to be used with bone cement.

Also if desired, solid additives (for example, tricalcium phosphate and/or hydroxyapatite) can be mixed with bone or such additives can separately be sprayed onto a prosthesis to be implanted into bone or onto a bone-bone juncture using the apparatus of the invention.

Fig. 1 is a plan view of an embodiment of the apparatus of the invention which is especially suitable for spraying moist bone particles (shown without a cover plate 78, as pictured in Fig. 2).

Fig. 2 is a plan view of the apparatus of Fig. 1 with a cover plate 78 (absent in Fig. 1) shown in place over the left portion of the device opposite the handle portion 12.

Fig. 3 is a plan rear view of the device of Fig. 1 showing a cartridge 36 (in which bone particles are packed and from which they are dispersed) in its mounted position on the spray gun body.

Fig. 4 is a plan view of a portion of the device of Fig. 3 but with the cartridge 36 (wherein bone particles are packed and from which they are dispersed) shown separated from the spray gun body 28.

Fig. 5 is a view partially in cross-section of the apparatus of Fig. 1 shown in its unactuated position, wherein the return spring 60 (shown in Fig. 1 but not in Fig. 5) is in its elongated, rest position and wherein a slice of the compressed bone particles 108 has not yet been moved upwardly into the path of the gas jet.

Fig. 6 is a view partially in cross-section of the apparatus of Fig. 1, in which the return spring 60 has been actuated by pulling the lever 70 to the right, the spring 60 is in its in its compressed position, and a slice of compressed bone particles 108 has been moved upwardly and into the path of the gas jet.

Fig. 7 is a cross-sectional view of the apparatus of Fig. 5, taken along the line 7-7.

Fig. 8 is a cross-sectional view of the apparatus of Fig. 6, taken along the line 8-8.

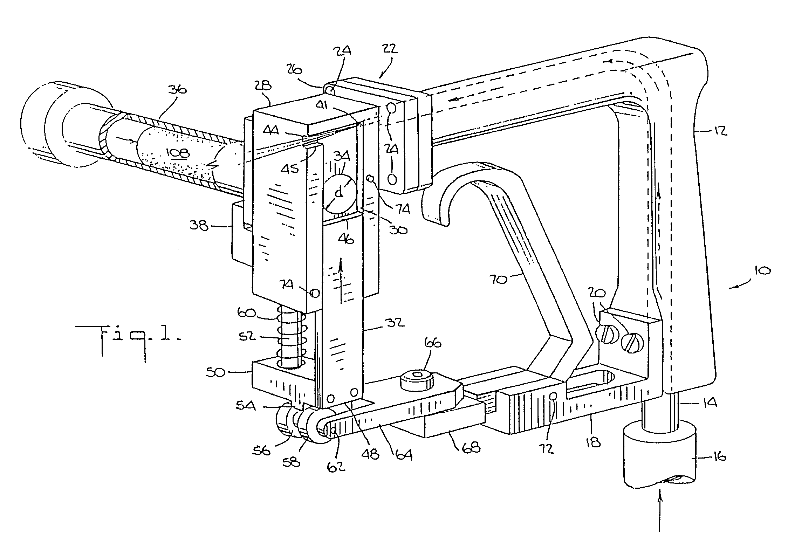

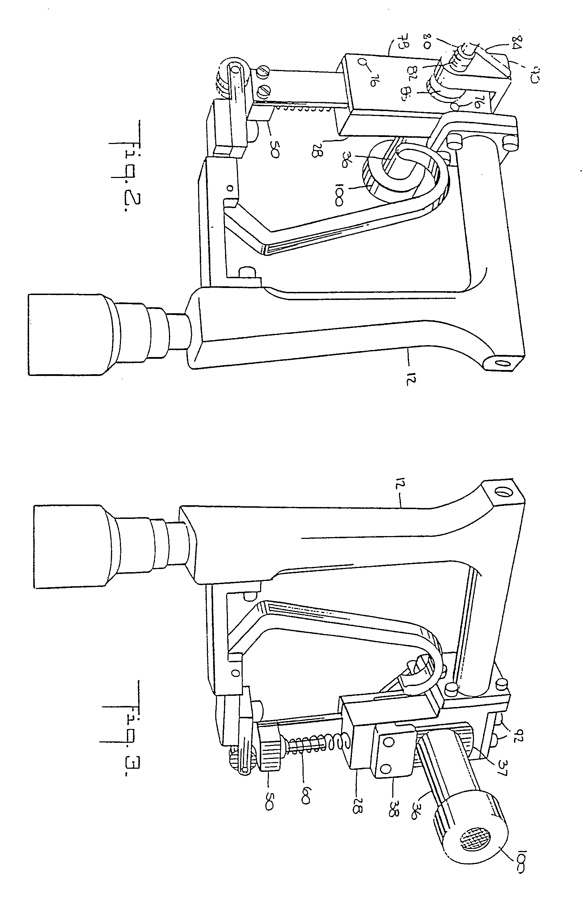

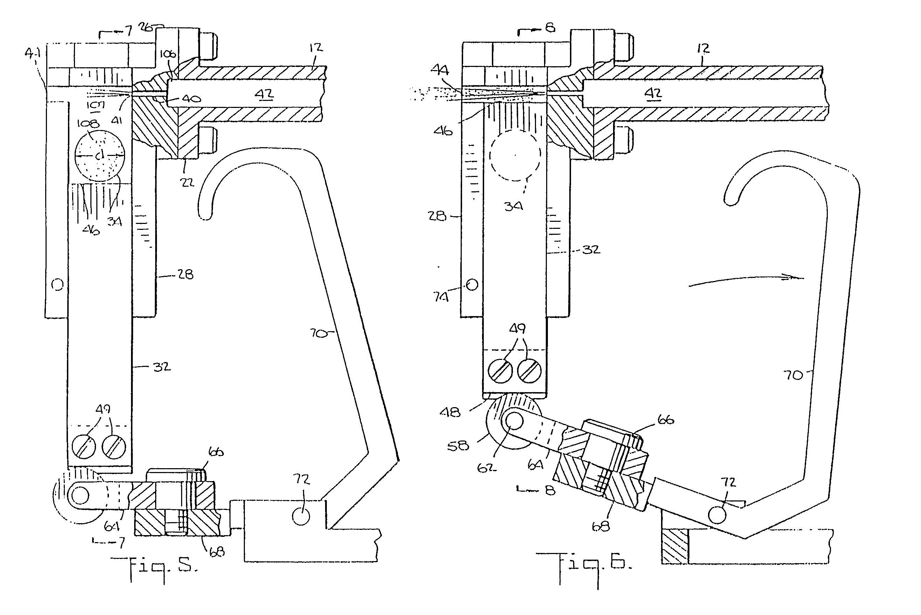

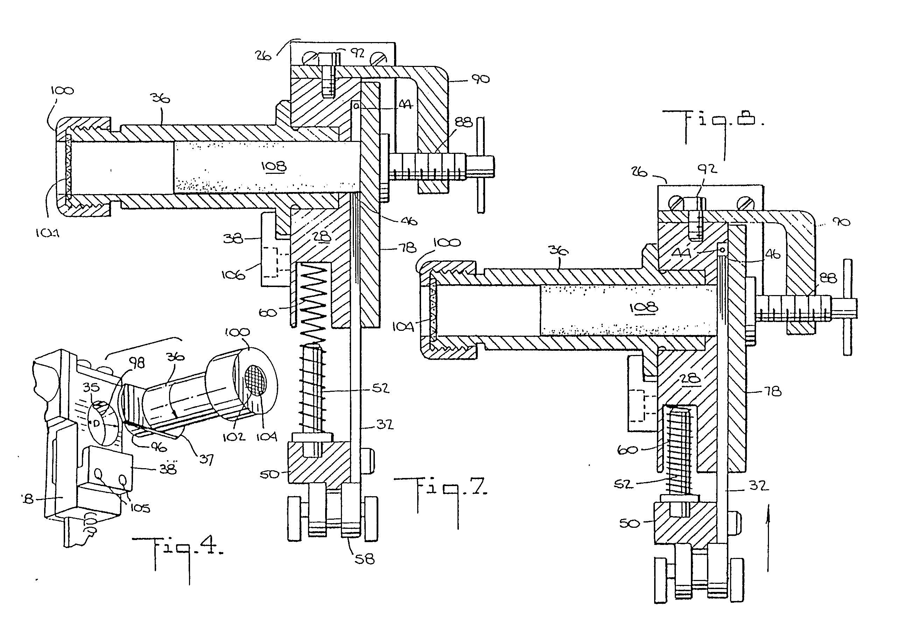

Referring to Fig. 1, bone spray gun (generally referred to as 10) is comprised of a hollow handle 12, through which compressed air passes. Other parts attached to handle 12 are a hose 14, to which an air hose adapter 16 is attached. A lever bracket 18 is attached to the bottom portion of hollow handle 12 by means of screws 20. At the top and left-hand extremity (in Fig. 1) of hollow handle 12, an adapter portion 22 is welded. Screws 24 (of which the end portions are shown in Fig. 1) are used to mount adapter portion 22 of hollow handle 12 onto adapter portion 26 of spray gun body 28. Adapter portion 26 is an integral part of spray gun body 28 (as shown more clearly in Figs. 3 and 4).

Within spray gun body 28, a passageway 30 is present, into which slide 32 fits snugly and along which slide 32 moves upwardly during activation of bone spray gun 10. Hollow bore 34 is also located within spray gun body 28. As shown in Fig. 1, hollow bore 34 has a diameter d; however, within spray gun body 28, hollow bore 34 is in open communication with hollow bore 35 (which has a diameter D larger than d) as shown in Fig. 4. Into diameter D, bone cartridge 36 (which has a diameter only slightly smaller than D) is positioned and locked into place on spray gun body 28 by a flange 37 which locks into cartridge retaining bracket 38 in a bayonet lock fashion.

As best shown in Fig. 5, spray gun body 28 has located therein a bored passageway 40, one end of which is air jet hole 41. Passageway 40 is in open communication with the left-hand portion of hollow handle 12, such that air or other suitable gas (described below) can pass from hollow handle 12, through spray gun body 28, and out of spray gun 10.

As best shown in Fig. 6, air jet hole 41 is substantially directly in line with bone spray exit hole 44, which also is substantially directly in line with the top 46 of slide 32 when slide 32 is in its uppermost and fully actuated position.

The bottom 48 of slide 32 is attached by means of screws 49 onto base 50 (see Fig. 1), to which a spring guide pin 52 is fixedly attached. Base 50 has on its bottom surface (as an integral part thereof) an appendage 54. Appendage 54 fits within a grove 56 of a lever roller bearing 58. Appendage 54 is maintained in its position within grove 56 by means of a return spring 60 and is maintained therein at all times, including when return spring 60 is in its uncompressed (i.e., rest) position and when return spring 60 is in its compressed (i.e., actuated) position.

Lever rolling bearing 58 is held in a fixed position with respect to adaptor portion 64 by means of a pivot pin 62 on adapter portion 64 of lever bracket 18.

Adapter portion 64 is held in a fixed position with respect to first portion 68 by means of screw 66 on first portion 68. First portion 68 and lever 70 can be integral or can be two parts fixedly attached together.

Lever 70 is attached to lever bracket 18 by means of lever pivot pin 72, about which lever 70 can rotate.

Referring to Figs. 1 and 2, small projections 74 (in Fig. 1) located on spray gun body 28 fit within bores 76 in cover 78 (in Fig. 2) and thereby help to hold cover 78 on spray gun body 28. Cover 78 is held in a fixed position on spray gun body 28 also by means of clamp 80 having a thread 82, a handle 84, and a clamp plate 86. When handle 84 is turned so that clamp plate 86 engages cover 78, cover 78 is held fast in place.

Thread 82 of clamp 80 passes through and is supported by bore 88 (shown in Figs. 7 and 8) in bracket 90. Bracket 90 is held in a fixed position by means of screws 92 (shown in Figs. 3, 7 and 8) on spray gun body 28.

Also shown in Fig. 3 in it assembled position is bone cartridge 36 (which has a diameter only slightly smaller than diameter D of bore 34 shown in Fig. 4.) Cartridge 36 has as an integral part thereof a flange 37. When cartridge 36 is inserted within diameter D of hollow bore 35, flange 37 rests flush and flat against spray gun body 28; and the extremity 96 (shown in Fig. 4) of cartridge 36 passes within hollow bore 35 until extremity 96 abuts against surface 98 (which forms the boundary between hollow bore 34 having diameter d and hollow bore 35 having diameter D). The other end 100 of cartridge 36 has a passage 102 with a screen 104 located thereover. Screen 104 is in open communication with the atmosphere.

Also shown in Fig. 4 is cartridge retaining bracket 38, which is held in place on spray gun body 28 by means of screws 105.

The parts described above are correspondingly labeled in all figures. Figs. 5-8 will now be described further with the operation of the apparatus.

Referring to Fig. 5, in operation, when lever 70 is in its rest position, air passes through hollow handle 12 (by way of hose 14 and air hose adapter 16 from an outside source of pressurized gas, not shown). The jet of gas proceeds into passageway 42 located within adapter portion 22 (which can be, if desired, an integral part of hollow handle 12 or more preferably can be welded onto hollow handle 12). The source of pressurized gas is preferably left on continuously. The gas proceeds through passageway 42 and into passageway 106, which is located within adapter portion 26. Passageway 106 is in open communication with passageway 40; and the other extremity of passageway 40 is air jet hole 41. The jet of air then enters into space 107, which is located between air jet hole 41 and bone spray gun exit hole 44. As shown in Fig. 5, when the bone spray gun 10 is not actuated, the top 46 of slide 32 is located below and does not cover hollow bore 34. For purposes of illustration, bone particles 108 are shown located within bore 34. In the apparatus shown in Fig. 5, the return spring 60 (although not shown in Fig. 5) is in its rest position.

Prior to the actuation of the bone spray gun 10, air proceeds out of bone spray gun exit hole 44 but the bone particles 108 remain within bore 34. In actual operation, cover 78 (not shown in Fig. 5 but shown in Fig. 2) presents the surfaces onto which bone particles 108 are forced by air coming into spray gun body 28 through screen 104 of bone cartridge 36, drawn in by the Venturi effect.

In Fig. 6, showing the bone spray gun 10 in its actuated position, lever 70 has been pulled to the right about pivot pin 72. Lever roller bearing 58 (which is in a fixed position with respect to lever 70) is forced in an upward direction and thereby also forces upwardly appendage 54 of base 50 (which base remains in contact with the lower extremity of return spring 60, not shown in Fig. 6 but shown in Figs. 7 and 8). The bottom 48 of slide 32 which is in contact with lever roller bearing 58 is also simultaneously forced upwardly. The top 46 of slide 32 then moves upwardly and slices off a portion of bone particles 108 which are located within hollow bore 34. This small sliced-off portion of bone particles 108 is then pushed upwardly by slide 32 into space 107 located between air jet hole 41 and bone spray exit hole 44. These particles are then forced out of the gun through exit hole 44 by the continuous air jet while bore 34 is covered by slide 32.

When lever 70 is released, spring 60 (shown in Figs. 7 and 8) then returns to its uncompressed, rest position. Then, more bone particles 108 are forced against cover 78; and in the next activation of lever 70, another slice of bone particles is sliced off by slide 32.

The metering capability of the above-described device is extremely important because it prevents clogging of the device by the bone particles. Also, the relatively small bone spray gun exit hole achieves a finely divided and controlled spray pattern of the bone particles.

The apparatus described above is suitable for metering other solids besides bone. For example, bone mixed with additives or additives alone (for example tricalcium phosphate and/or hydroxyapatite) can be metered and sprayed with this device in the method of the invention onto a prosthesis or bone-bone juncture. The invention also includes a method for manufacturing a prosthesis, said method comprising spraying particles of either living bone or other bone having a growth factor onto said prosthesis, wherein the largest dimension of said particles lies within the range from 100 microns to 1.5 mm, and wherein said spraying is done and all other treatments of said bone are done at a temperature which is low enough and in an atmosphere which is suitable such that said bone particles remain alive or at least retain their growth factor.

## Claims

1. An apparatus 10 suitable for spraying a measured amount of relatively wet solid particles, characterized in that said apparatus 10 comprises in operable communication the following items:

(a) a housing 28 having a first aperture 44 located within said housing for allowing a gas jet to exit from said housing 28;

(b) means for holding 36 a relatively large amount of said particles 108 at a second aperture 34 spaced apart from said first aperture 44, wherein intake air passes through said relatively large amount of said particles 108 and into said housing 28 when said second aperture 34 is in open communication with the atmosphere and is in an open position;

(c) means for separating off 32 a relatively small amount of said particles from said relatively large amount of said particles 108; and

(d) means for placing 70, 58, 60, 32 said relatively small amount of said particles and said first aperture 44 into proximity with one another, such that said second aperture 34 is in a closed position and is no longer in open communication with the atmosphere.

2. An apparatus 10 according to claim 1, characterized in that said relatively wet particles have a largest dimension which lies within the range from 100 microns to 1.5 mm and wherein said means for separating off 32 a small amount of particles comprises a means for slicing 46.

3. An apparatus 10 according to claim 2, characterized in that said means for placing 70, 58, 60, 32 said separated small amount of particles and said first aperture 44 into proximity with one another comprises a spring 60 for moving said separated small amount of particles into the path of said gas jet at said first aperture 44.

4. An apparatus according to claim 3 and including also a handle 12 for holding said apparatus 10 and through which handle 12 said gas jet can pass.

5. An apparatus according to claim 4, characterized in that said handle 12 comprises also a means for actuating 70, 58 said means for separating off a small amount of particles 32.

6. An apparatus according to claim 5, characterized in that said handle 12 also contains a means for actuating 70, 58 said spring 60 so as to move said separated amount of particles into the path of said gas jet at said first aperture 44.

7. An apparatus according to claim 6, characterized in that said means for holding 36 is a cartridge 36 into which said particles are packed.

8. A method for spraying a prosthesis to improve bone ingrowth into, and for stimulating living bone attachment to, said prosthesis, said method comprising spraying particles of either living bone or other bone having a growth factor onto said prosthesis, wherein the largest dimension of said particles lies within the range from 100 microns to 1.5 mm, and wherein said spraying is done and all other treatments of said bone are done at a temperature which is low enough and in an atmosphere which is suitable such that said bone particles remain alive or at least retain their growth factor.

9. A method according to claim 8, wherein said temperature of said bone particles remains within the range from 50 to 100° F and wherein said atmosphere comprises clean and filtered compressed air.

10. A method according to claim 9, wherein said temperature is about 70° F.

11. A method according to claim 10, wherein the pressure of said compressed air is within the range from 30 to 80 p.s.i.

12. A prosthesis suitable for implantation into a human body comprising a biocompatible material, the surface of which prosthesis has adhered thereto particles of living bone or other bone particles which retain a growth factor, which have been sprayed onto said prosthesis, and which have a largest dimension which is within the range from 100 microns to 1·5 mm.

13. A prosthesis according to claim 12 wherein said bone particles are living bone.

PC 7548

Fig.1.

Fig.2.

Fig.3.

PC 7548

Fig. 6.

Fig. 5.

Fig.8.

Fig.7.

Fig.4.

EP 0 382 457 A2

PC 7548